# EUROPEAN PATENT APPLICATION

(11) **EP 1 862 465 A1**
(43) Date of publication of application: **05.12.2007**
(21) Application number: 06729566.7
(22) Date of filing: 20.03.2006
(51) Int. Cl.: C07F 7/18, C07C 45/27, C07C 47/19, C07B 53/00

(54) **METHOD OF PRODUCING 3-HYDROXYBUTYRALDEHYDE DERIVATIVE**

(30) Priority: 23.03.2005 JP 2005084430
(71) Applicant: Kaneka Corporation, Kita-ku Osaka-shi, Osaka 530-8288 (JP)
(72) Inventor: IZUMIDA, Masashi, o, Takasago-shi, Hyogo, 6768688 (JP); MATSUMOTO, Shingo, o, Takasago-shi, Hyogo, 6768688 (JP)
(74) Representative: Vossius & Partner
(86) International application number: PCT/JP2006/305598
(87) International publication number: WO 2006/101092

(57) **Abstract**

The present invention relates to a method of producing a 3-hydroxybutyraldehyde derivative, particularly an optically active 3-hydroxybutyraldehyde derivative, which is useful as an intermediate of a pharmaceutical agent and the like.

The method of the present invention is **characterized by** converting the 3-hydroxybutyrate derivative to an alcohol derivative by a reduction reaction followed by converting it to a desired aldehyde derivative by an oxidation reaction using a nitroxyl compound and a co-oxidant. In accordance with the present invention, a 3-hydroxybutyraldehyde derivative having high qualities (a low content of alcohol derivatives) can be produced by an easy and convenient method without resorting to a reaction under an extremely low temperature condition.

## Description

### Technical Field

The present invention relates to a 3-hydroxybutyraldehyde derivative, particularly to an optically active 3-hydroxybutyraldehyde derivative which is useful as an intermediate of a pharmaceutical agent and the like.

### Background Art

A 3-hydroxybutyraldehyde derivative, in particular, an optically active (3R)-tert-butyldimethylsilyloxybutyraldehyde is a useful compound as an intermediate of a pharmaceutical agent and the like. With respect to a method of production thereof, following methods are known.
(1) A method reducing optically active (3R)-methyl (tert-butyldimethylsilyloxy)-butyrate by diisobutylaluminum hydride under a condition at -80 °C (Patent Document 1);
(2) A method reducing optically active (3R)-methyl (tert-butyldimethylsilyloxy)-butyrate by diisobutylaluminum hydride under a condition at -78 °C (Nonpatent Literature 1);
(3) A method reducing optically active (3R)-methyl (tert-butyldimethylsilyloxy)-butyrate by a reducing agent comprising sodium bis-(2-methoxyethoxy)aluminum hydride and amines under a condition from -40 °C to a room temperature (Patent Document 2);
(4) A method of production by subjecting an alcohol derivative to Swern oxidation under a condition at -78 °C, and the alcohol derivative is obtained by reducing optically active (3R) -methyl (tert-butyldimethylsilyloxy)-butyrate by diisobutylaluminum hydride under a condition at a room temperature (Nonpatent Literature 2); and
(5) A method of production by oxidizing 3-methyl (tert-butyldimethylsilyloxy)-butanol using a chromium oxide (Nonpatent Literature 3).

In a conversion from an ester derivative to an aldehyde derivative by a reducing agent such as the above (1) and (2), in order to suppress an alcohol derivative which is a by-product generated by over-reduction of the aldehyde derivative, a temperature needs to be strictly controlled at an extremely low temperature. However, in a production at an industrial scale, a special equipment such as ultralow-temperature equipment is required; therefore, these production methods cannot be insisted as versatile methods.

On the other hand, according to the method (3), it is possible to implement the production at a room temperature by improving the reducing agent, but a great amount of an alcohol derivative is generated as a by-product by over-reduction of the desired aldehyde derivative, and thus there have been some points of improvement in terms of quality of desired products. Additionally, since sodium bis-(2-methoxyethoxy)aluminum hydride used as a reducing agent is expensive, from an economic viewpoint, there are some points to be improved in usage of the agent for industrial production of (3R)-tert-butyldimethylsilyloxybutyraldehyde.

The method (4) is a method converting an alcohol derivative obtained by reduction reaction to the desired aldehyde derivative by oxidation reaction. Therefore, the alcohol derivative which is a problem in the (1), (2) and (3) is to be a raw material for the oxidation reaction so that controlling a content thereof is easy, but with respect to a condition for the oxidation reaction, an extremely low temperature of -78 °C is necessary. Further, due to a use of oxalyl chloride which is expensive as an oxidant and which is a corrosive substance, and due to a generation of dimethyl sulfide as a by-product having a strong odor and the like in the reaction, it cannot be insisted as a versatile production method in terms of safety and good environment in addition to generality of equipment.

Since the method (5) uses chromium oxide as an oxidant, it cannot be insisted as a versatile production method in production at an industrial scale in terms of good environment.

As described above, previous methods have a problem in terms of industrial production, for example, that they require an extreme low temperature condition. If the extremely low temperature condition is avoided, there remains a problem, for example, that a content of the alcohol derivative becomes higher and a quality of the desired product becomes lowered. A industrially suitable production method which avoids the extremely low temperature condition and is capable of providing an desired product having a low content of alcohol derivatives has not been established.
Patent Document 1: Japanese unexamined patent publication No. 63-233989
Patent Document 2: Japanese unexamined patent publication No. 2-290887
Nonpatent Literature 1: Journal of the American Chemical Society, 1990, 112, 7079 to 7081
Nonpatent Literature 2: Helvetica Chimica Acta, 1989, 72, 165 to 171
Nonpatent Literature 3: Helvetica Chimica Acta, 1981, 64, 1467

### Disclosure of Invention

### Problems to be Solved by Invention

The present invention provides a versatile production method of 3-hydroxybutyraldehyde derivative, in particular an optically active 3-hydroxybutyraldehyde derivative, which is useful as an intermediate of a pharmaceutical agent; the method can avoid an extremely low temperature condition and provide a desired product having a low content of an alcohol derivative.

### Means of Solving the Problems

The present inventors have intensively studied the above problems, and completed the method of producing an optically active 3-hydroxybutyraldehyde derivative by converting a 3-hydroxybutyrate derivative to an alcohol derivative by a reduction reaction under a room temperature condition using sodium borohydride, aluminum chloride and the like followed by converting it to a desired aldehyde derivative by an oxidation reaction using, for example, a nitroxyl compound such as 4-hydroxy-2,2,6,6-tetramethylpiperidine-1-oxyl and a co-oxidant such as an aqueous solution of sodium hypochlorite under an ice-cold temperature condition. The method can avoid an extremely low temperature condition (for example, -60 °C or lower) and provides a desired product having a low content of alcohol derivatives.

Namely, the present invention relates to a method of producing a 3-hydroxybutyraldehyde derivative represented by general formula (1), comprising
reducing a 3-hydroxybutyrate derivative represented by general formula (2) using a reducing agent to obtain a 3-hydroxybutanol derivative represented by general formula (3), and
oxidizing the 3-hydroxybutanol derivative thus obtained with a nitroxyl compound represented by general formula (4) and a co-oxidant: 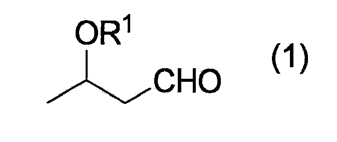 wherein R¹ represents hydrogen or a protecting group of a hydroxyl group; 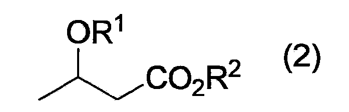 wherein R¹ is same as described above; R² represents an alkyl group having 1 to 20 carbon atom(s) which may have a substituent, an aralkyl group having 7 to 20 carbon atoms which may have a substituent, or an aryl group having 6 to 20 carbon atoms which may have a substituent; 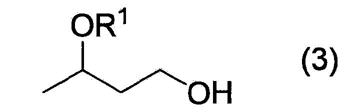 wherein R¹ is same as described above; and 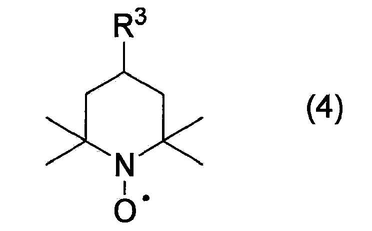 wherein R³ represents hydrogen, an alkyl group having 1 to 20 carbon atom(s) which may have a substituent, an aralkyl group having 7 to 20 carbon atoms which may have a substituent, an aryl group having 6 to 20 carbon atoms which may have a substituent, a hydroxyl group, an alkoxyl group having 1 to 10 carbon atom(s) which may have a substituent, an acyl group having 1 to 10 carbon atom(s) which may have a substituent, or an amino group which may have a substituent.

### Effects of the Invention

In accordance with the present invention, a 3-hydroxybutyraldehyde derivative having a high quality (having a low content of an alcohol derivative) can be produced by a versatile production method avoiding an extremely low temperature condition.

### Description of the Preferred Embodiment

Hereinafter, the present invention is further explained.
The 3-hydroxybutyraldehyde derivative produced in the present invention is a compound represented by general formula (1) :

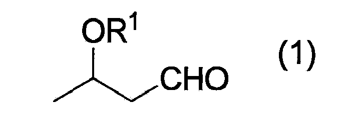

In formula (1), R¹ represents hydrogen, or a protecting group of a hydroxyl group. Here, the protecting group of the hydroxyl group is not particularly limited, but specifically includes a silyl-based protecting group, an ether-based protecting group, an acetal-based protecting group, or an ester-based protecting group.

The silyl-based protecting group means a group capable of forming a silyloxy bond for protecting the hydroxyl group, which may include a trimethylsilyl group, a triethylsilyl group, a tert-butyldimethylsilyl group, and a tert-butyldiphenylsilyl group.

The ether-based protecting group means a group capable of forming an ether bond for protecting the hydroxyl group, which may include a methyl group, an ethyl group, a tert-butyl group, an octyl group, an allyl group, a benzyl group, a p-methoxymethyl group, a fluorenyl group, a trityl group, and a benzhydryl group.

The acetal-based protecting group represents a group capable of forming an acetal bond for protecting the hydroxyl group, which may include, for example, a methoxyethyl group, an ethoxyethyl group, a tetrahydropyranyl group, and a tetrahydrofuranyl group.

The ester-based protecting group represents a group capable of forming an ester bond for protecting a hydroxyl group, which may include an acetyl group, a propionyl group, an isopropionyl group, a pivaloyl group, a benzoyl group, a trifluoroacetyl group, and a trichloroacetyl group.

Among them, R¹ is preferably hydrogen, the silyl-based protecting group, the acetal-based protecting group, the ester-based protecting group, more preferably the silyl-based protecting group, the acetal-based protecting group, and the ester-based protecting group. The silyl-based protecting group is even more preferably a tert-butyldimethylsilyl group, and the acetal-based protecting group is even more preferably a tetrahydropyranyl group, and the ester-based protecting group is even more preferably a pivaloyl group. In particular, the silyl-based protecting group is preferred, and among them, a tert-butyldimethylsilyl group is particularly preferred.

Next, the 3-hydroxybutyrate derivative used as a starting material of the present invention is a compound represented by general formula (2):

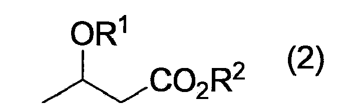

In formula (2), R¹ is the same as described above.

R² represents an alkyl group having 1 to 20 carbon atom(s) which may have a substituent, an aralkyl group having 7 to 20 carbon atoms which may have a substituent, or an aryl group having 6 to 20 carbon atoms which may have a substituent. The alkyl group, the aralkyl group and the aryl group may be unsubstituted or may have a substituent. The substituent may include an amino group, a hydroxyl group, a phenyl group, an aryl group, an alkanoyl group, an alkenyl group, an alkynyl group, an alkoxyl group, a nitro group, a halogen atom and the like.

The alkyl group having 1 to 20carbon atom(s) which may have a substituent is not particularly limited, and it may be, for example, a linear alkyl group such as a methyl group, an ethyl group, a n-propyl group, a n-butyl group, a n-pentyl group; or a branched alkyl group such as an isopropyl group, an isobutyl group, a tert-butyl group, a neopentyl group, and a tert-pentyl group. The aralkyl group having 7 to 20 carbon atoms which may have a substituent includes, for example, a benzyl group, a p-methoxybenzyl group, a phenethyl group, and a naphthylmethyl group. The aryl group having 6 to 20 carbon atoms which may have a substituent includes, for example, a phenyl group and a naphthyl group.

Among them, R² is preferably the alkyl group having 1 to 20 carbon atom(s) which may have a substituent, and even more preferably the methyl group or the ethyl group, particularly preferably the methyl group.

Next, a method of producing the 3-hydroxybutanol derivative represented by general formula (3), namely a reduction reaction is explained:

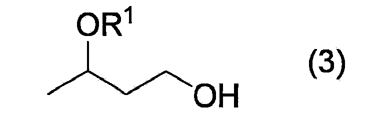

An explanation of R¹, specific examples, and preferred examples in formula (3) are same as described above.

In the present invention, the reducing agent used for the reduction reaction is not particularly limited as long as it reduces the 3-hydroxybutyrate derivative into the 3-hydroxybutanol derivative, and for example, it may include one described in Journal of the American Chemical Society, 1990, 112, 7079 to 7081; specifically, it may include an alkaline metal, a aluminum hydride compound, a boron hydride compound, and a silicon hydride compound.

The alkaline metal is not particularly limited, and may include lithium, sodium, potassium and the like.

The aluminum hydride compound is not particularly limited, and may be, for example, aluminum hydride which may have a substituent, an alkali metal aluminum hydride which may have a substituent and the like.

The aluminum hydride which may have a substituent may include an aluminum hydride, and monosubstituted or disubstituted aluminum hydride. The substituent herein is not particularly limited, and may include an alkyl group having 1 to 4 carbon atom(s), a phenyl group, an aryl group, an alkanoyl group, an alkenyl group, an alkynyl group, a hydroxyl group, an alkoxyl group, a nitro group, an amino group, and a halogen atom. The alkyl group is not particularly limited, and for example, may be a linear alkyl group such as a methyl group, an ethyl group and a n-propyl group; or a branched alkyl group such as an isopropyl group, an isobutyl group, and a tert-butyl group.

The alkali metal aluminum hydride which may have a substituent may include an alkali metal aluminum hydride, and monosubstituted or disubstituted alkali metal aluminum hydride. The alkaline metal is not particularly limited, and may include lithium, sodium, potassium and the like. The substituent here may be a same substituent of the aluminum hydride.

The boron hydride compound is not particularly limited, and may include a boron hydride which may have a substituent and a metal borohydride compound which may have a substituent.

The boron hydride which may have a substituent may include boron hydride, and monosubstituted or disubstituted boron hydride. The substituent here is not particularly limited, and may be an alkyl group having 1 to 20 carbon atom(s), a phenyl group, an aryl group, an alkanoyl group, an alkenyl group, an alkynyl group, a hydroxyl group, an alkoxyl group, a nitro group, an amino group, and a halogen atom. The alkyl group is not particularly limited, and may be a linear alkyl group such as a methyl group, an ethyl group and a n-propyl group or a branched alkyl group such as an isopropyl group, an isobutyl group, and a tert-butyl group.

The metal borohydride compound which may have a substituent may include alkali metal boron hydride, alkaline-earth metal boron hydride, zinc boron hydride and the like, and a monosubstituted, a disubstituted, or a trisubstituted compound of them. The alkaline metal is not particularly limited, and may include lithium, sodium, potassium and the like. The alkaline-earth metal is not particularly limited and may include beryllium, magnesium, calcium and the like. The substituent here may include the same substituent of the boron hydride.

The silicon hydride compound is not particularly limited, and may include, for example, silicon hydride which may have a substituent.

The silicon hydride which may have a substituent may include silicon hydride, and monosubstituted, disubstituted, or a trisubstituted silicon hydride. The substituent is not particularly limited, and may include the same substituent of the aluminum hydride.

Among them, in the present invention, the reducing agent is preferably an aluminum hydride compound or a boron hydride compound.

Specifically, as the aluminum hydride compound, diisobutylaluminum hydride, lithium aluminum hydride, and sodium methoxyethoxyaluminum hydride are more preferred, and diisobutylaluminum hydride is particularly preferred.

As the boron hydride compound, boron hydride, sodium borohydride, and lithium borohydride are more preferred, and sodium borohydride is particularly preferred.

In particular, a boron hydride compound is preferred.

An amount of the reducing agent to be used is not particularly limited as long as it is 2. 0 molar equivalents of hydrido or more relative to the 3-hydroxybutyrate derivative, but is preferably 2 to 20 times molar equivalent, more preferably 2 to 10 times molar equivalent, and in view of safety in order not to generate an excessive amount of hydrogen, it is particularly preferred to use 2 to 5 times molar equivalent.

Further, in the present invention, the reduction reaction is preferably carried out under a coexistence of a reaction accelerator. The reaction accelerator is not particularly limited as long as it promotes the reduction reaction, and may include, for example, an alcohol, boron halide, a metal halide, metal sulfate or the like.

The alcohol is not particularly limited, and may include, for example, an alcohol which may have a substituent. Specifically, it may include methyl alcohol, ethyl alcohol, n-propyl alcohol, isopropyl alcohol, isobutyl alcohol, sec-butyl alcohol, tert-butyl alcohol and the like. The substituent in a case of having a substituent may include an amino group, a hydroxyl group, a phenyl group, an aryl group, an alkanoyl group, an alkenyl group, an alkynyl group, an alkoxyl group, a nitro group, a halogen atom and the like.

The halogen atom of the boron halide or the metal halide is not particularly limited, and may include, for example, fluorine, chlorine, bromine, iodine and the like.

The metal of the metal halide or the metal sulfate are not particularly limited, and may include, for example, iron, copper, aluminum, zinc, cobalt and the like in addition to an alkaline metal such as lithium, sodium, and potassium, an alkaline-earth metal such as beryllium, magnesium, and calcium.

Among them, the alkyl alcohol having 1 to 4 carbon atom (s) which may have a substituent or the metal halide is preferred. As the alkyl alcohol having 1 to 4 carbon atom (s), methyl alcohol or ethyl alcohol is more preferred, and as the metal halide, an alkaline metal halide or an aluminum halide is more preferred. Among them, in particular, lithium chloride or aluminum chloride is particularly preferred.

An amount of the reaction accelerator to be used is not particularly limited as long as it can promote the reduction reaction. It is, for example, 0.001 to 10 times mol, preferably 0.01 to 5 times mol, more preferably 0.1 to 3 times mol as an amount of the reaction accelerator relative to 3-hydroxybutyrate derivative represented by formula (2).

The solvent in the reduction reaction is not particularly limited, and may include water, an organic solvent, or a mixture thereof. The organic solvent may include an alcohol, an ether, a nitrile, a hydrocarbon, and an ester solvent.

The alcohol solvent is not particularly limited, and may include, for example, methyl alcohol, ethyl alcohol, n-propyl alcohol, isopropyl alcohol, isobutyl alcohol, sec-butyl alcohol, tert-butyl alcohol and the like, and it is preferably the methyl alcohol or the ethyl alcohol. When the alcohol solvent is used as the solvent, the solvent alcohol itself can be used as the reaction accelerator.

The ether solvent is not particularly limited, and may include, for example, diethyl ether, diisopropyl ether, tetrahydrofuran, 1,4-dioxane, 1,2-dimethoxyethane, diethylene glycol dimethyl ether and the like, and it is preferably 1,2-dimethoxyethane, or diethylene glycol dimethyl ether.

The nitrile solvent is not particularly limited, and may include, for example acetonitrile and propionitrile, preferably acetonitrile.

The hydrocarbon organic solvent is not particularly limited, and may include, for example, toluene, benzene, xylene, hexane, cyclohexane, and heptane, preferably toluene from an economic view point.

The ester solvent is not particularly limited, and may include, for example, methyl acetate, ethyl acetate, propyl acetate, methyl propionate and ethyl propionate, preferably the ethyl acetate.

The organic solvent may be used alone or as a mixture of two or more thereof. Among them, in particular, the alcohol solvent, the ether solvent, a mixture of the alcohol solvent and the ether solvent, or a mixture of the ether solvent and the hydrocarbon solvent are preferred, even more preferred are the ether solvent, or the mixture of the ether solvent and the hydrocarbon solvent.

When the mixture solvent of the ether solvent and the hydrocarbon solvent is used, in view of reaction time and a cost of the solvent, a mixture of 1,2-dimethoxyethane and toluene or a mixture of diethylene glycol dimethyl ether and toluene are particularly preferred.

A mixture ratio of the ether solvent and the toluene is not particularly limited, but a ratio of the ether solvent to toluene is preferably 1:0 to 0:1, more preferably 1:0 to 1:1, particularly preferably 1:0 to 4:1 in weight ratio.

A concentration in feed of a substrate is not particularly limited, but a feeding is normally carried out in a range that a concentration of the 3-hydroxybutyrate derivative is from 0.5 to 70 % by weight. The feeding can be suitably carried out preferably at a concentration of 1 to 70 % by weight, more preferably 5 to 60 % by weight, particularly 10 to 60 % by weight. If the concentration is too low, volumetric efficiency becomes poor, so that it is inefficient.

A method of feeding is not particularly limited, and an order of adding the 3-hydroxybutyrate derivative represented by formula (2), the reducing agent and the solvent is not particularly limited. In a case of using the reaction accelerator, an order of addition is not particularly limited. Usually, a method adding the compound represented by formula (2) to the solvent followed by adding the reducing agent and adding the reaction accelerator as necessary is used.

A reaction temperature is not particularly limited, but it is normally 100 °C or lower, preferably 80 °C or lower, more preferably 60 °C or lower, even more preferably 40 °C or lower, particularly 30 °C or lower. A lower limit may be any temperature as long as it does not prevent a progress of the reaction, and is normally 0 °C or higher, preferably 5 °C or higher. Normally, the reaction can be carried out suitably around 20 °C.

The resultant 3-hydroxybutanol derivative in reaction liquid may be directly supplied to a next step, or may be separated or purified by a common separation method, for example, extraction, washing, condensation, distillation, column chromatography or the like, or a combination thereof.

For example, a reaction completed liquid containing the 3-hydroxybutanol derivative may be directly used in a next step, or may be purified to be used in the next step. In a case of purification, for example, the compound can be obtained by washing an organic layer with an acid aqueous solution and subsequently washing with a basic solution followed by distillation, but a method of obtaining the compound is not limited to the methods.

The 3-hydroxybutanol derivative obtained by the above method can be converted into the desired 3-hydroxybutyraldehyde derivative (1) by oxidizing a hydroxyl group. Next, a method of production of the 3-hydroxybutyraldehyde derivative represented by formula (1), namely an oxidation reaction is explained.

The nitroxyl compound used for the oxidation reaction is represented by general formula (4):

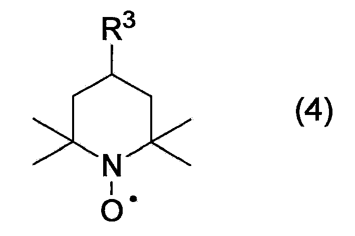

In formula (4), R³ represents hydrogen, an alkyl group having 1 to 20 carbon atom(s) which may have a substituent, an aralkyl group having 7 to 20 carbon atoms which may have a substituent, an aryl group having 6 to 20 carbon atoms which may have a substituent, a hydroxyl group, an alkoxyl group having 1 to 10 carbon atom(s) which may have a substituent, an acyl group having 1 to 10 carbon atom(s) which may have a substituent, or an amino group which may have a substituent. These groups may be unsubstituted or may have a substituent. The substituent may include an amino group, a hydroxyl group, a phenyl group, an aryl group, an alkanoyl group, an alkenyl group, an alkynyl group, an alkoxyl group, a nitro group, a halogen atom and the like.

The alkyl group having 1 to 20 carbon atom(s) which may have a substituent is not particularly limited, and may be, for example, a linear alkyl group such as a methyl group, an ethyl group, and an n-propyl group or a branched alkyl group such as an isopropyl group, an isobutyl group, a tert-butyl group, a neopentyl group, and a tert-pentyl group.

The aralkyl group having 7 to 20 carbon atoms which may have a substituent may include, for example, a benzyl group, a p-methoxy benzyl group, a phenethyl group, and a naphthylmethyl group. The aryl group having 6 to 20 carbon atoms which may have a substituent is, for example, a phenyl group and a naphthyl group.

The alkoxyl group having 1 to 10 carbon atom (s) which may have a substituent may include, for example, a linear alkoxyl group such as a methoxy group, an ethoxy group, an n-propyloxy group or a branched alkyl group such as an isopropyl group, an isobutyl group, a tert-butyl group, a neopentyl group, and a tert-pentyl group.

The acyl group having 1 to 10 carbon atom(s) which may have a substituent, for example, is a linear acyl group such as a formyl group, an acetyl group, and an n-propionyl group, or a branched alkyl group such as a pivaloyl group.

Among them, in particular, R³ is preferably hydrogen, the hydroxyl group, the alkoxyl group having 1 to 10 carbon atom(s), or the acyl group having 1 to 10 carbon atom(s), more preferably hydrogen or the hydroxyl group, particularly preferably the hydroxyl group.

An amount of the nitroxyl compound to be used is not particularly limited. It is 0.0001 to 5 times mol, preferably 0.001 to 1 times mol, more preferably 0.005 to 0.5 times mol, and normally 0.01 to 0.1 times mol as an amount of nitroxyl compound relative to the 3-hydroxybutanol derivative used for the reaction.

The oxidation reaction can be normally carried out under any of a basic condition, a neutral condition or an acidic condition, and in particular, it is preferably carried out under the basic condition or the neutral condition, most preferably under the basic condition. Specifically, it is preferably carried out at pH 7. 0 or more. However, if the oxidation reaction is carried out with a solvent system other than a solvent system containing water, the condition is not limited to the above.

In adjusting pH, for example, there can be a method using an acid compound, a method using a basic compound or a method adding an inorganic salt and the like, but the method is not limited to these.

In the oxidation reaction of the present invention, a co-oxidant is used together with the nitroxyl compound. The co-oxidant used for the oxidation reaction is not particularly limited as long as it can oxidize the nitroxyl compound, for example, a hypohalite, a halite, a N-halogen succinimide, a perbenzoic acid which may have a substituent, an organic periodinane and a solution containing them.

The co-oxidant may play a role of oxidizing the nitroxyl compound, and other than using the co-oxidant, a method by electrode oxidation is also included as a method of the present invention.

Halogen of hypohalite, halite, or N-halogen succinimide is not particularly limited, and may include, for example, fluorine, chlorine, bromine, iodine and the like, preferably chlorine, or bromine, more preferably chlorine.

A counterion of hypohalite or halite is not particularly limited, and may include, for example, an alkaline metal, an alkaline-earth metal and the like.

The alkaline metal of the salt may include, for example, lithium, sodium, potassium and the like, preferably sodium.

The alkaline-earth metal of the salt may include, for example, beryllium, magnesium, calcium and the like, preferably calcium.

The perbenzoic acid which may have a substituent may include, for example, perbenzoic acid, and monosubstituted or disubstituted perbenzoic acid.

The substituent is not particularly limited, and may include an alkyl group having 1 to 4 carbon atom(s), a phenyl group, an aryl group, an alkanoyl group, an alkenyl group, an alkynyl group, a hydroxyl group, an alkoxyl group, a nitro group, an amino group, a halogen atom and the like.

The alkyl group is not particularly limited, and may be, for example, a linear alkyl group such as a methyl group, an ethyl group, and an n-propyl group, or a branched alkyl group such as an isopropyl group, an isobutyl group, and a tert-butyl group.

The organic periodinane may include iodosylbenzene, iodosobenzene diacetate, iodoxybenzene, Dess-Martin Periodinane and the like, preferably the Dess-Martin Periodinane.

Among them, from an economic view point, the hypohalite and the halite is more preferred as the co-oxidant. In particular, the hypohalite is even more preferred.

As the hypohalite, sodium hypochlorite, calcium hypochlorite is particularly preferred.

The co-oxidant is not particularly limited, and may be used alone or in combination, or as a solution thereof.

The solvent of the solution is not particularly limited as long as it dissolves the co-oxidant, and may be water, an organic solvent, or a mixture thereof.
The organic solvent may include a hydrocarbon, an alcohol, an ether, and an ester, which may be used alone or as a mixture of two or more.

Among them, from an economic view point, water or the hydrocarbon solvent is particularly preferred. As the hydrocarbon organic solvent, toluene is preferred.

An amount of the co-oxidant to be used is not particularly limited as long as it is an equivalent or more relative to the 3-hydroxybutanol derivative to be used, and it is 1 to 5 times mol, preferably 1 to 3 times mol, more preferably 1 to 2 times mol, particularly preferably 1 to 1.5 times mol as equivalent of the co-oxidant relative to the 3-hydroxybutanol derivative to be used for the reaction.

The solvent used for the oxidation reaction is not particularly limited, and may include, for example, water, an organic solvent, or a mixture system thereof. The organic solvent may include a solvent such as an ether, a nitrile, a hydrocarbon, and an ester solvent. In the reaction, a solvent is preferably used, but it is also allowed to carry out the reaction without using a solvent.

The ether solvent is not particularly limited, and may include, for example, diethyl ether, diisopropyl ether, tetrahydrofuran, 1,4-dioxane, 1,2-dimethoxyethane, and diethylene glycol dimethyl ether, preferably tetrahydrofuran.

The nitrile solvent is not particularly limited, and may include, for example, acetonitrile and propionitrile, preferably acetonitrile.

The hydrocarbon organic solvent is not particularly limited, and may include, for example, toluene, benzene, xylene, hexane, cyclohexane, heptane and the like, preferably toluene from an economic view point.

The ester solvent is not particularly limited and may include, for example, methyl acetate, ethyl acetate, propyl acetate, methyl propionate, ethyl propionate and the like, preferably the ethyl acetate.

The organic solvent may be used alone or as a mixture of two or more. Among them, in particular, from an economic view point, toluene or water is preferred, and a mixture of toluene and water is more preferable.

A mixture ratio of toluene and water is not particularly limited, and preferably, a ratio of the toluene to water is 1:0 to 1:50, more preferably 1:0 to 1:10, particularly 1:0 to 1:5 in weight ratio.

A concentration in feed of the substrate is not particularly limited, but a feeding is normally carried out in a range that a concentration of the 3-hydroxybutanol derivative becomes 0.5 to 50 % by weight. It is preferably 1 to 40 % by weight, more preferably 5 to 30 % by weight, particularly 5 to 15 % by weight. If the concentration is too low, volumetric efficiency becomes poor, resulting in a lack of efficiency.

A method of feeding is not particularly limited, and may include, for example, (1) a method adding a nitroxyl compound and a co-oxidant to a 3-hydroxybutanol derivative under in-solvent or non-solvent condition, followed by adjusting pH; (2) a method adding a nitroxyl compound to a 3-hydroxybutanol derivative under in-solvent or non-solvent condition, followed by adjusting pH and, subsequently, adding a co-oxidant; (3) a method adding adjusting pH of 3-hydroxybutanol derivative under in-solvent or non-solvent condition, followed by adding a nitroxyl compound and a co-oxidant; and (4) a method in accordance with the above (1) to (3) without adjusting pH. It is suitably carried out normally by method (1).

A temperature during the reaction is not particularly limited, but normally the reaction is carried out at 100 °C or lower, preferably 80 °C or lower, more preferably 60 °C or lower, even more preferably 40 °C or lower, particularly preferably 30 °C or lower. A lower limit is not particularly limited, but it is, for example, -20 °C or higher, preferably-10 °C or higher, more preferably -5 °C or higher. Normally, the reaction can be carried out suitably under an ice-cold temperature condition.

Thus generated 3-hydroxybutyraldehyde derivative in reaction liquid may be directly supplied to a next step as necessary, or may be separated or purified by a common separation method, for example, extraction, washing, condensation, distillation, column chromatography or the like, or a combination thereof.

For example, if the 3-hydroxybutanol derivative is used as the substrate for the oxidation reaction along with base and an aqueous solution of sodium hypochlorite under in-solvent or non-solvent condition in a presence of the nitroxyl compound, a reaction completed liquid containing the 3-hydroxybutyraldehyde derivative may be directly used in a next step, or may be used in a next step after purification, too. In a case of carrying out the purification, for example, the compound can be obtained by removing an aqueous layer and subsequently washing an organic layer with water followed by distillation, but a method of obtaining the compound is not limited to the methods.

The 3-hydroxybutyraldehyde derivative can be suitably produced by the above methods under a temperature condition at -10 °C or higher.

Further, the 3-hydroxybutyraldehyde derivative obtained by the present invention has an extremely high quality. In particular, a content of the 3-hydroxybutanol derivative in a product material can be suppressed without requiring the extremely low temperature condition, although the extremely low temperature condition has conventionally been needed to suppress the content of the 3-hydroxybutanol derivative. A content of the 3-hydroxybutanol derivative in an end product obtained by the process of the present invention is to be 10 % or less, particularly 5 % or less.

In accordance with the present invention, a 3-hydroxybutyraldehyde derivative having a high quality (having a low content of alcohol derivatives) can be produced by a versatile production method since it does not require an extremely low temperature condition.

The producing method of the present invention also preferably applies to optical active material 3-hydroxybutyraldehyde derivative. In such a case, a corresponding optically active 3-hydroxybutyrate derivative may be used as a starting material. The optical active material here may be either R-type or S-type, and may also be a mixture thereof wherein one of them is present in excess.

Further, the 3-hydroxybutyraldehyde derivative obtained by the above method can be converted to a 3-hydroxy buten-1-yl silyl ether derivative useful as an intermediate of a pharmaceutical agent and the like represented by general formula (5):

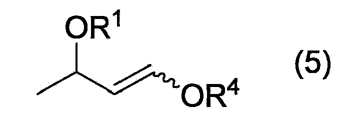

In formula (5), R⁴ represents a silyl-based protecting group. The silyl-based protecting group means a group capable of forming a silyloxy bond for protecting a hydroxyl group, and may include a trimethylsilyl group, a triethylsilyl group, a tert-butyldimethylsilyl group, and a tert-butyldiphenylsilyl group.

Among them, in particular, the silyl-based protecting group which is preferred as R⁴ includes a trimethylsilyl group, and a tert-butyldimethylsilyl group, in particular, the trimethylsilyl group is particularly preferred.

A method for conversion to the 3-hydroxybuten-1-yl silyl ether derivative is not particularly limited, but may include a method of reaction with a silylation agent, for example, a method described in Japanese unexamined patent publication No. 63-233989 wherein amines and silyl halide are made to act.

### EXAMPLES

Hereinafter, the present invention is described in more detail by Examples, but the present invention is not restricted by the following Examples.

In Examples, a quantitative determination of (3R)-tert-butyldimethylsilyloxybutanol in a reaction product was carried out using a gas chromatography (column: OV-17 5 % Chromosorb WAW DMCS 80/100 mesh (ID 3 mm x 2 m; manufactured by GL Sciences Inc.), column temperature: 100 °C, 25 minutes, rate of heating: 10 °C/minute, final temperature: 300 °C, 5 minutes, carrier gas: nitrogen, flow rate adjusted so that a retention time of the (3R)-tert-butyldimethylsilyloxybutanol becomes around 17 minutes; detection: FID).

### (Example 1)

75.0 g of (3R)-methyl (tert-butyldimethylsilyloxy) -butyrate was mixed with 21. 8 g of 1, 2-dimethoxyethane, and 9.8 g of sodium borohydride was added thereto. Then 17.2 g of aluminum chloride was added so as to maintain the mixture at a temperature of 40 °C or lower, and the mixture was stirred for 40 hours. 149.0 g of toluene was added to a reaction liquid to cool to 15 °C or lower. Into 214.0 g of 10 % by weight of hydrochloric acid aqueous solution, a reaction completed liquid was added to maintain a temperature at 15 °C or lower. After an aqueous layer was removed, an organic layer was washed with 200.0 g of water to obtain 243.9 g of toluene solution of (3R)-tert-butyldimethylsilyloxybutanol (net weight (purity): 63.7 g, reduction reaction yield: 98 %).

18.6 g of the toluene solution (net weight (purity): 5.0 g) of the (3R)-tert-butyldimethylsilyloxybutanol was mixed with 0.1 g of the 4-hydroxy-2,2,6,6-tetramethylpiperidine-1-oxyl and 21.9 g of an aqueous solution pf sodium hypochlorite (effective chlorine concentration: 10 wt %), and the mixture was cooled with ice bath. Next, 0.2 g of sodium hydrogen carbonate was added, and the mixture was stirred for 1 hour, and an aqueous layer was disposed of and an organic layer was cleaned with 22 g of water to obtain 17.6 g of toluene solution of (3R)-tert-butyldimethylsilyloxybutyraldehyde (net weight (purity) : 4.4 g, oxidation reaction yield: 88 %). The reaction product contained 0.9 mol % of the (3R)-tert-butyldimethylsilyloxybutanol.

### (Example 2)

100.0 g of (3R)-methyl (tert-butyldimethylsilyloxy) -butyrate was mixed with 57. 0 g of diethylene glycol dimethyl ether and 197.0 g of toluene, and 12.9 g of sodium borohydride was added thereto. 46.2 g of aluminum chloride was added so as to maintain the mixture at a temperature of 40 °C or lower, and the mixture was stirred for 72 hours and then cooled to 15 °C or lower. Into 234.0 g of 5 % by weight hydrochloric acid, a reaction completed liquid was added so as to maintain a temperature at 15 °C or lower. After an aqueous layer was removed, an organic layer was washed with 106.0 g of an aqueous solution of dilute potassium hydroxide to obtain 321.0 g of a toluene solution of (3R)-tert-butyldimethylsilyloxybutanol (net weight (purity): 86.3 g, reduction reaction yield: 100 %).

### (Example 3)

5.0 g of (3R) -methyl (tert-butyldimethylsilyloxy)-butyrate was mixed with 7.8 g of tetrahydrofuran and 1.2 g of sodium borohydride. Next, 1.4 g of lithium chloride and 2.8 g of methanol were added thereto and the mixture was stirred at 50 °C for 7 hours. Analysis of the reaction liquid showed that 0.9 g of (3R)-tert-butyldimethylsilyloxybutanol (reduction reaction yield: 20 %) had been obtained.

### (Example 4)

Into 74.2 g of a toluene solution of (3R)-tert-butyldimethylsilyloxybutanol (net weight (purity): 20.0 g), 0.5 g of 4-hydroxy-2,2,6,6-tetramethylpiperidine-1-oxyl and 2.1 g of sodium hydrogen carbonate were added and the mixture was cooled with ice bath. After 21.9 g of an aqueous solution of sodium hypochlorite (effective chlorine concentration: 10 wt %) was added thereto while keeping a temperature at 10 °C or lower, and a mixture was stirred for 30 minutes. After an aqueous layer was removed, an organic layer was washed with 80.0g of water. The aqueous layer was removed to obtain 17.3 g of a toluene solution of (3R)-tert-butyldimethylsilyloxybutyraldehyde (net weight (purity) : 16.1 g, yield: 81 %). The reaction product contained 0.3 mol % of (3R)-tert-butyldimethylsilyloxybutanol.

### (Example 5)

Into 5.2 g of (3R)-tert-butyldimethylsilyloxybutanol (net weight (purity): 5.0 g), 0.08 g of 4-hydroxy-2,2,6,6-tetramethylpiperidine-1-oxyl was added, and the mixture was cooled with ice bath. After 28. 3g of calcium hypochlorite (effective chlorine concentration: 63 wt %) was added thereto, and the mixture was stirred for 41 hours, and then filtered. Filtrate was washed with toluene to obtain 58.4 g of a toluene solution of (3R)-tert-butyldimethylsilyloxybutyraldehyde (net weight (purity): 3.4g, yield: 70 %). The reaction product contained 1.6 mol % of (3R)-tert-butyldimethylsilyloxybutanol.

### (Example 6)

Into 46.4 g of a toluene solution of (3R)-tert-butyldimethylsilyloxybutyraldehyde (net weight (purity): 16.7 g), 20.9 g of triethylamine and 16.2 g of trimethylsilyl chloride were added, and the mixture was stirred for 20 hours under a reflux condition. After a reaction liquid was cooled to a room temperature, the liquid was filtered and a filtrate was washed with toluene. Next, after the filtrate was purified by simple distillation, it was subj ected to washing with 5 % acid saline (pH2) and then washing with 5 % saline to obtain 16.8 g of (3R)-tert-butyldimethylsilyloxy buten-1-yl trimethylsilyl ether (net weight (purity): 16.5 g, yield: 72.7 %).

### (Comparative Example 1)

Into 2.5 ml of a toluene solution of 2.3 M sodium bis-(2-methoxyethoxy)aluminum hydride, 0.96 ml of di-n-propylamine was slowly added under nitrogen atmosphere and was stirred for 1.5 hours to prepare a reducing agent. Into a solution obtained by dissolving 0.95 g of (3R)-methyl (tert-butyldimethylsilyloxy)-butyrate in 2.0 ml of toluene, the reducing agent thus prepared was slowly dropped at a room temperature. From an analysis of the reaction liquid after about 3 hours, it was found that a yield of (3R)-tert-butyldimethylsilyloxybutyraldehyde toluene was 80.9 % and the reaction liquid contained 6.7 mol % of (3R)-tert-butyldimethylsilyloxybutanol.

## Claims

1. A method of producing a 3-hydroxybutyraldehyde derivative represented by general formula (1), comprising
reducing a 3-hydroxybutylate derivative represented by general formula (2) using a reducing agent to obtain a 3-hydroxybutanol derivative represented by general formula (3), and
oxidizing the obtained 3-hydroxybutanol derivative using a nitroxyl compound represented by general formula (4)and a co-oxidant: 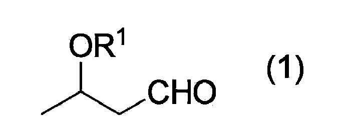 wherein R¹ represents hydrogen, or a protecting group of a hydroxyl group; 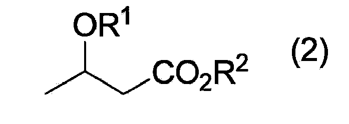 wherein R¹ is the same as described above; R² represents an alkyl group having 1 to 20 carbon atom (s) which may have a substituent, an aralkyl group having 7 to 20 carbon atoms which may have a substituent, or an aryl group having 6 to 20 carbon atoms which may have a substituent; 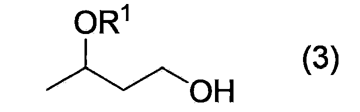 wherein R¹ is the same as described above; and 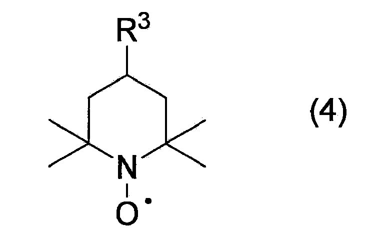 wherein R³ represents hydrogen, an alkyl group having 1 to 20 carbon atom(s) which may have a substituent, an aralkyl group having 7 to 20 carbon atoms which may have a substituent, an aryl group having 6 to 20 carbon atoms which may have a substituent, hydroxyl group, an alkoxyl group having 1 to 10 carbon atom(s) which may have a substituent, an acyl group having 1 to 10 carbon atom(s) which may have a substituent, or an amino group which may have a substituent.

2. The method of production according to claim 1 wherein the reduction is carried out under a coexistence of a reaction accelerator.

3. The method of production according to claim 1 or 2, wherein the reducing agent is an aluminum hydride compound, or a boron hydride compound.

4. The method of production according to any one of claims 1 to 3, wherein the reducing agent is a boron hydride compound.

5. The method of production according to any one of claims 2 to 4, wherein the reaction accelerator is alkyl alcohol having 1 to 4 carbon atom(s) which may have a substituent or a metal halide.

6. The method of production according to claim 5, wherein the reaction accelerator is methyl alcohol or ethyl alcohol.

7. The method of production according to claim 5, wherein the reaction accelerator is an alkali metal halide or an aluminum halide.

8. The method of production according to any one of claims 1 to 7, wherein R³ of the nitroxyl compound represented by formula (4) is hydrogen, a hydroxyl group, an alkoxyl group having 1 to 10 carbon atom(s) which may have a substituent, or an acyl group having 1 to 10 carbon atom(s) which may have a substituent.

9. The method of production according to claim 8, wherein R³ is hydrogen or a hydroxyl group.

10. The method of production according to any one of claims 1 to 9, wherein the oxidizing 3-hydroxybutanol derivative represented by formula (3) is carried out under a neutral or a basic condition.

11. The method of production according to any one of claims 1 to 10, wherein the co-oxidant is a compound or a solution thereof which is capable of oxidizing the nitroxyl compound represented by formula (4).

12. The method of production according to any one of claims 1 to 11, wherein the co-oxidant is hypohalite or halite.

13. The method of production according to any one of claims 1 to 12, wherein the co-oxidant is hypohalite.

14. The method of production according to any one of claims 1 to 13, wherein R¹ is a silyl-based protecting group.

15. The method of production according to any one of claims 1 to 14, wherein the 3-hydroxybutyraldehyde derivative represented by formula (1) is an optical active material.

16. The method of production according to any one of claims 1 to 15, wherein, in all steps, reactions are carried out at a reaction temperature of -10 °C or higher.

17. A method of producing a 3-hydroxybuten-1-yl silyl ether derivative represented by general formula (5) by reacting a 3-hydroxybutyraldehyde derivative obtained by a method of production according to any one of claims 1 to 16 with a silylation agent; 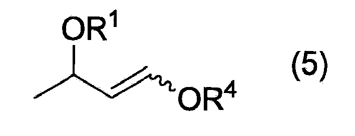 wherein R⁴ represents a silyl-based protecting group.
